# EUROPEAN PATENT APPLICATION

(11) **EP 4 443 441 A1**
(43) Date of publication of application: **09.10.2024**
(21) Application number: 24163165.4
(22) Date of filing: 13.03.2024
(51) Int. Cl.: G16H 50/30

(54) **SYSTEMS AND METHODS FOR CLASSIFYING WAVEFORM DATA USING AN AI MODEL**

(30) Priority: 04.04.2023 US 202318295785
(71) Applicant: GE Precision Healthcare LLC, Waukesha, WI 53188 (US)
(72) Inventor: PARDASANI, Rohit, 560066 Bengaluru (IN); AJITH, Siddharth, 560066 Bengaluru (IN); JORDAN, John Michael, Severna Park, 21146 (US); VITULLO, Renee L., Chapel, 33543 (US); JANAS, Merrinda, Phoenix, 85045 (US)
(74) Representative: Kilburn & Strode LLP

(57) **Abstract**

The current disclosure provides methods and systems to process waveform data. In one example, a method for a waveform processing system comprises displaying, on a display device of the waveform processing system (718), a location in a stream of waveform data of a segment including a specified event, wherein the location of the segment is identified based on aggregating outputs of an AI model (710) performing sliding window inferences on the stream of waveform data. Performing the sliding window inferences on the stream of waveform data may include dividing the waveform data into a plurality of overlapping chunks (706), each chunk comprising consecutive data values extracted from the stream of waveform data over a specified duration, and inputting each chunk into the AI model (708) to generate arrays of output values indicating a presence or absence the segment, which may then be aggregated.

## Description

### TECHNICAL FIELD

Embodiments of the subject matter disclosed herein relate to processing waveform data, and more particularly, to waveform segment identification using an artificial intelligence (AI) model.

### BACKGROUND

In a hospital environment, various monitors may generate clinical waveform data which may be reviewed by a healthcare provider, including fetal monitor data, electrocardiogram (ECG) data, electroencephalogram (EEG) data, intensive care unit (ICU) patient monitoring data, electromyography (EMG) data, and other types of waveform data. To aid the healthcare provider in reviewing the waveform data, one or more AI models may be trained to analyze the waveform data. For example, the healthcare provider may wish to know a fetal heart rate baseline over a 15 minute interval at a specified time of day, or a number of contractions that occurred between a first time and a second time of the day.

An AI model may be trained to identify segments of the waveform data where an event occurs, where the event may be an aberration or variation in the waveform data. If a duration of the waveform data is short, the waveform data may be collected and inputted into the AI model, and the AI model may output an indication of where a segment including the event is located within the waveform data. If the duration of the waveform data is ongoing or long, the waveform data may be inputted into the AI model in chunks, and the AI model may output an indication of where a segment including the event is located within a relevant chunk of waveform data. The outputs may then be aggregated across the chunks to determine the locations of the segments within the waveform data. A summary of the aggregated outputs may be generated periodically, which may be used to display the locations of the segments on a display device. However, a performance of the AI model may depend on how the outputs aggregated. Current methods for aggregating the outputs may result in a lower-than-desired performance of the AI model.

### SUMMARY

The current disclosure includes a waveform processing system, comprising a patient monitor configured to provide a stream of waveform data; a display device; a processor; and a non-transitory memory including instructions that when executed, cause the processor to control the display device to display a location of a segment including a specified event in a stream of waveform data on the display device based on a segment identification array, the segment identification array generated by dividing the waveform data into a plurality of overlapping chunks of a fixed length, where each chunk is offset from a preceding chunk by a predefined inference interval; at each inference interval, inputting a plurality of data values of each overlapping chunk of the waveform data into an artificial intelligence (AI) model, the AI model trained to output a chunk segment identification array including a respective plurality of output values, each output value of the respective plurality of output values indicating a presence or absence of a specified event in the overlapping chunk of the waveform data; and aggregating a plurality of chunk segment identification arrays outputted by the AI model for a corresponding plurality of overlapping chunks to create the segment identification array. In this way, the AI model performs sliding window inferences on the stream of waveform data.

In some embodiments, aggregating the outputs may include calculating an average of individual, time-aligned outputs of the current overlapping chunks, and assigning a one or a zero as a single aggregate output at a plurality of time references, depending on whether the average exceeds a threshold output value. Thus, the aggregated segment array may comprise a series of ones and zeros, where a consecutive number of ones may indicate a segment including the specified event, and a consecutive number of zeros may indicate a segment not including the specified event. Because the output values of the aggregated segment array have a 1:1 correspondence with the data values of the stream of waveform data, a starting position and an ending position of the consecutive number of ones may be used to determine a corresponding starting position and ending position of the segment in the stream of waveform data, for displaying on the display device.

By aggregating the outputs of the AI model across a plurality of overlapping chunks of data in this fashion, an accuracy of the aggregated output may be increased with respect to other approaches to aggregation, leading to more precise and reliable identification of segments of the waveform data including the specified event than currently achieved by the other aggregation approaches.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a block diagram of an exemplary embodiment of an waveform processing system configured to classify segments of waveform data, in accordance with an embodiment of the present disclosure;
FIG. 2A shows an exemplary training system for a waveform classification network, in accordance with an embodiment of the present disclosure;
FIG. 2B shows a block diagram of a set of processes for identifying a segment of waveform data within which an event has occurred using a waveform classification network, in accordance with an embodiment of the present disclosure;
FIG. 3 shows a codification of waveform data to indicate locations of specified events in the waveform data, in accordance with an embodiment of the present disclosure;
FIG. 4A is a diagram that shows how waveform data may be divided into chunks of a fixed length, in accordance with an embodiment of the present disclosure;
FIG. 4B is a diagram that shows inputs and outputs of a waveform classification network for identifying segments in the chunks, in accordance with an embodiment of the present disclosure;
FIG. 4C is a diagram that shows how outputs of the waveform classification network may be aggregated across a plurality of overlapping chunks, in accordance with an embodiment of the present disclosure;
FIG. 5A is a diagram showing a first step of a process for aggregating outputs of the waveform classification network, in accordance with an embodiment of the present disclosure;
FIG. 5B is a diagram showing a second step of the process for aggregating outputs of the waveform classification network, in accordance with an embodiment of the present disclosure;
FIG. 5C is a diagram showing a third step of the process for aggregating outputs of the waveform classification network, in accordance with an embodiment of the present disclosure;
FIG. 6 shows a flowchart illustrating an exemplary method for training the waveform classification neural network, in accordance with an embodiment of the present disclosure; and
FIG. 7 shows a flowchart illustrating an exemplary method for aggregating outputs of the waveform classification neural network to indicate locations of segments of raw waveform data including the specified events, in accordance with an embodiment of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

### DETAILED DESCRIPTION

Methods and systems are provided herein for processing waveform data using an AI model, such as a neural network model. In some examples, the AI model may perform a regression to classify a predetermined amount of the waveform data. In other examples, the AI model may be trained to predict a point in time when an event might occur, where the event may be an aberration or variation in the waveform data. This could be a region of peak, trough, or continued lack of expected variation for an extended period of time. The waveform data may be inputted into the AI model, and the AI model may output the predicted point in time. Alternatively, the AI model may be trained to identify segments of the waveform data where the event occurs. The waveform data may be inputted into the AI model, and the AI model may output an indication of where segments including the event are located within the waveform data.

Because the waveform data may be a continuous stream of data, or the predetermined amount of waveform data may have a duration that is too long to input into the AI model at once, the AI model may be trained on portions, described herein as chunks, of waveform data of a fixed length. The AI model may learn to identify one or more segments where an event occurs within each chunk of waveform data. The durations or lengths of each segment may vary, and the AI model may detect the segments based on different amounts of neighboring data of the waveform data. After training, the AI model may perform inferences on chunks of waveform data.

At regular inference intervals, the AI model may output results of the AI model for chunks that have been analyzed by the AI model. The outputs may be aggregated to generate a determination of where an event may have occurred within a longer duration of the waveform data (e.g., greater than the size of a chunk). An accuracy of the determination may depend on how the outputs are aggregated. To address an inadequacy of previous approaches to aggregating the outputs, the inventors herein propose a novel aggregation strategy that may be more robust than the previous approaches, and which may increase an accuracy of event detection.

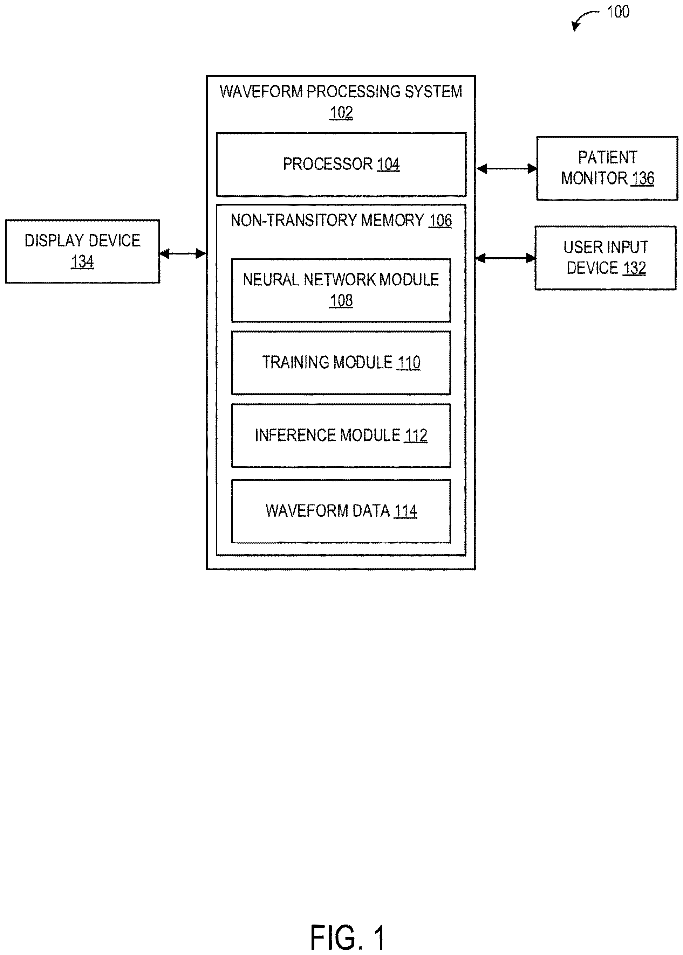

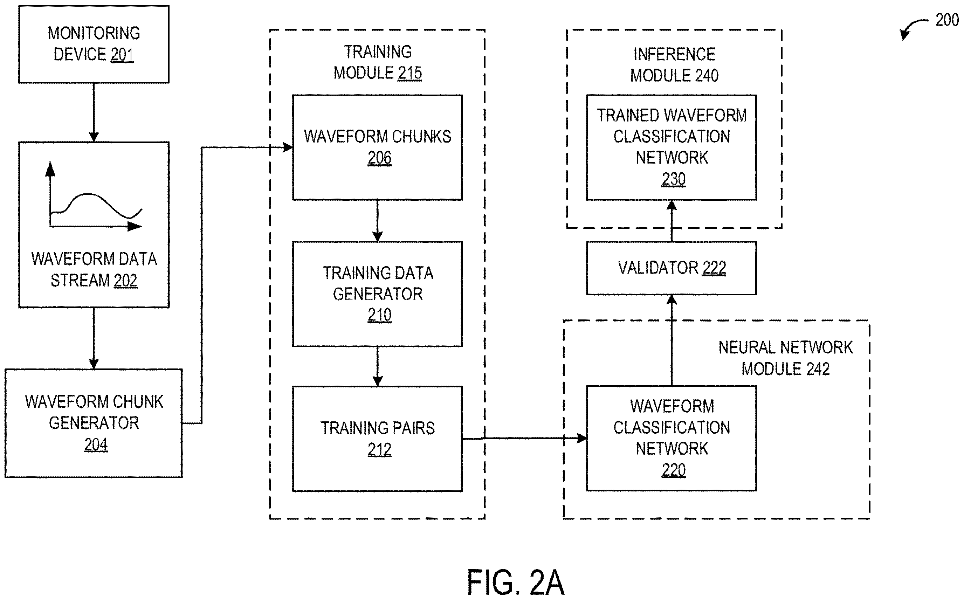

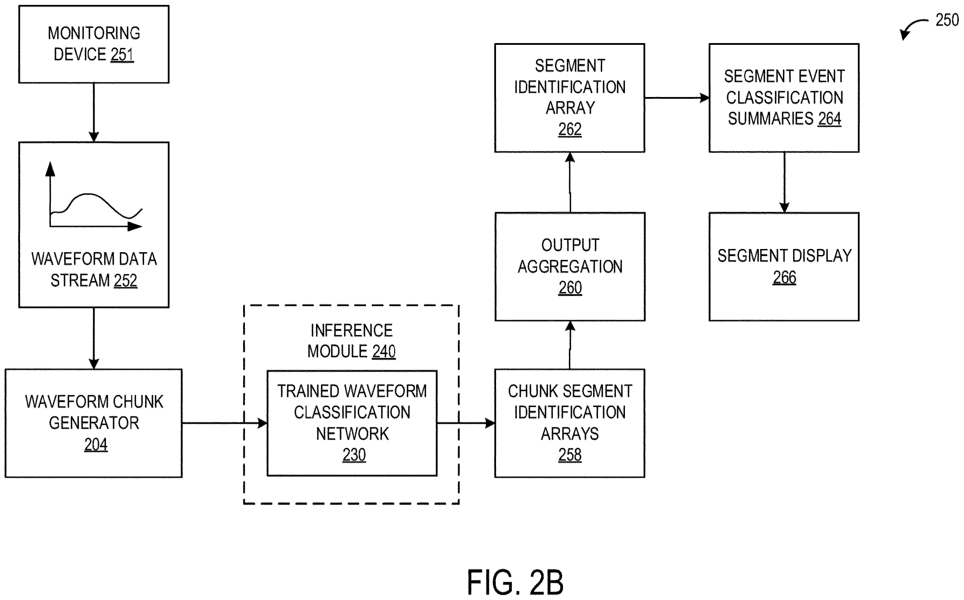

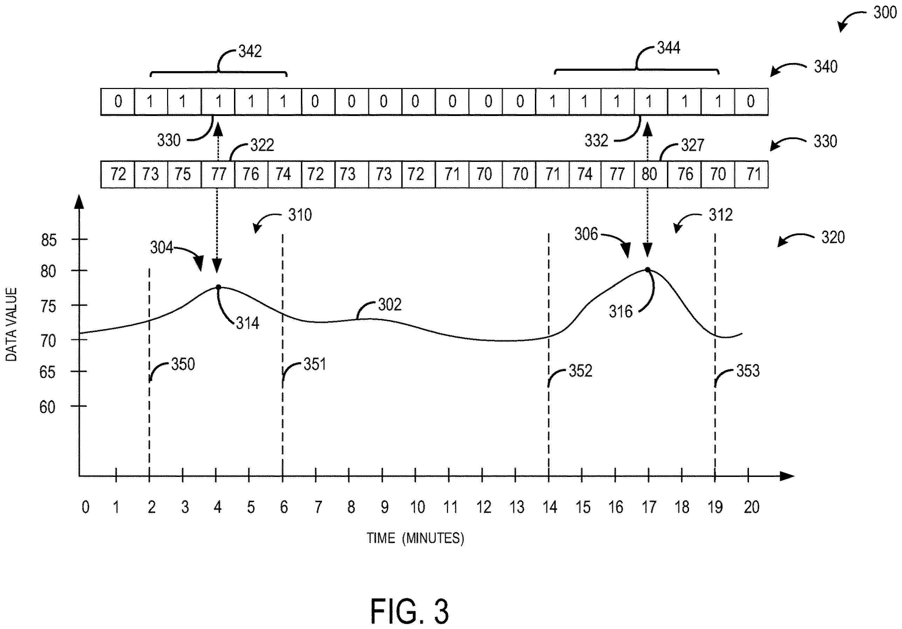

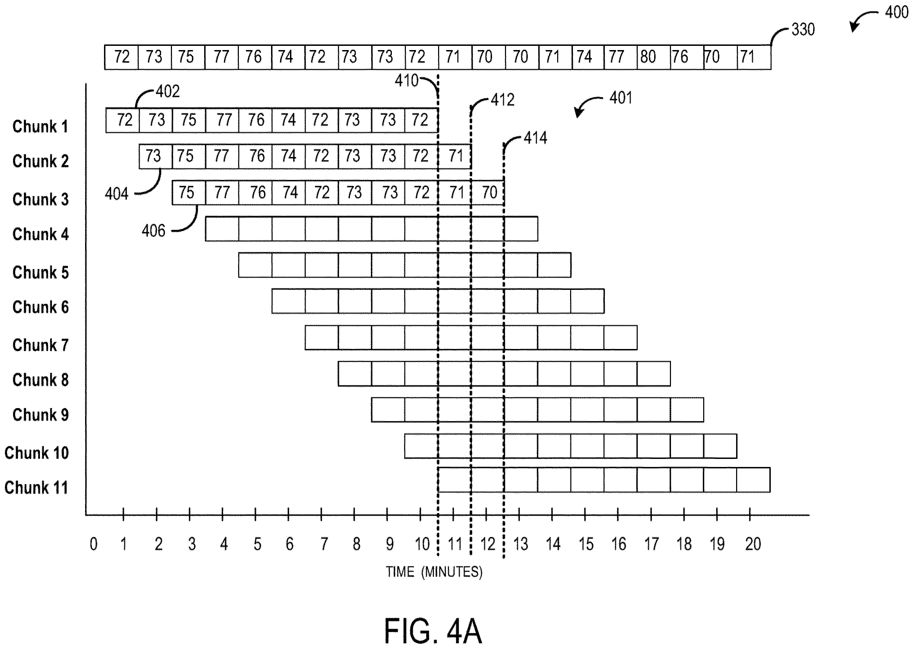

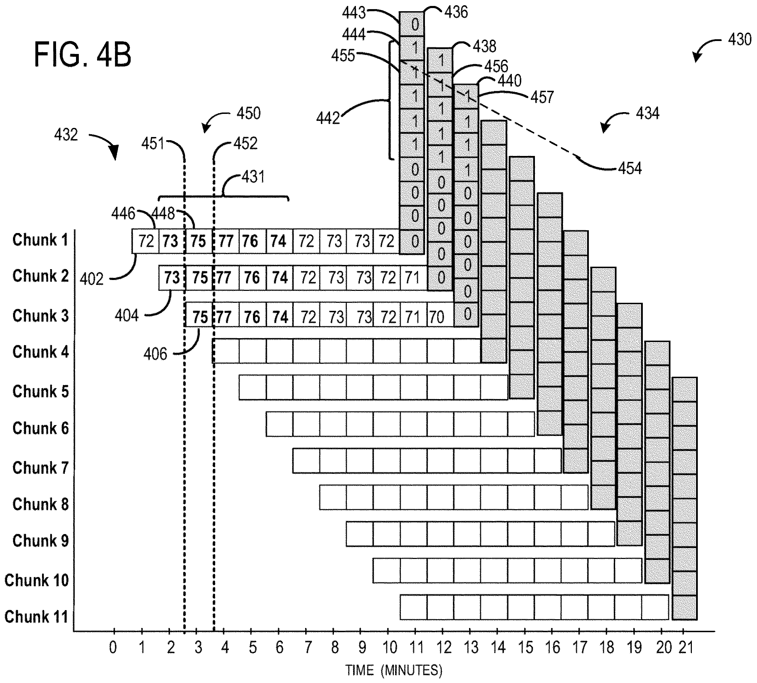

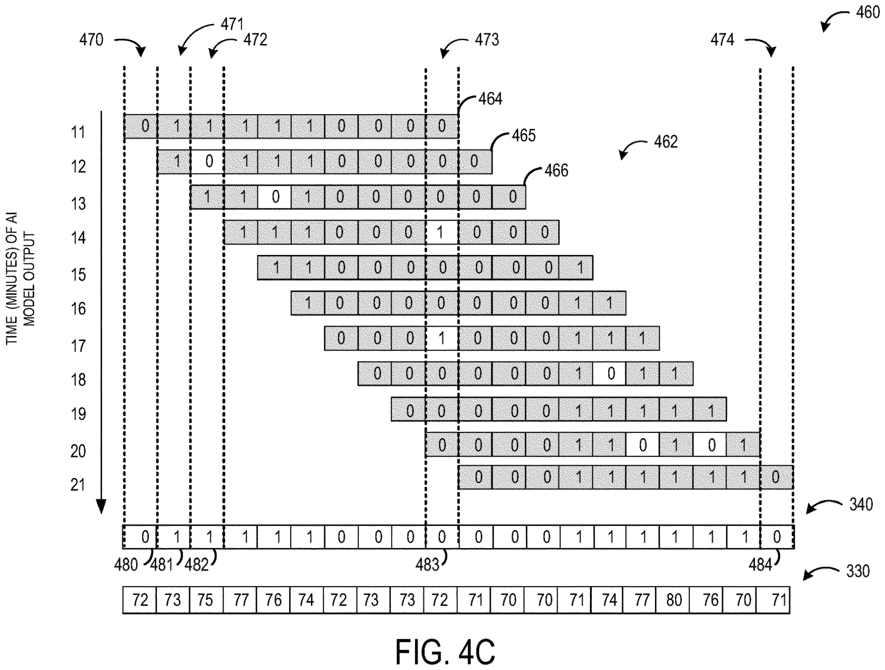

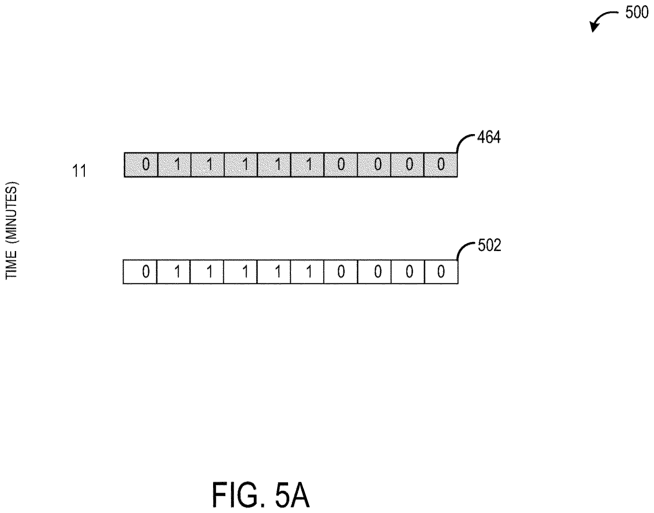

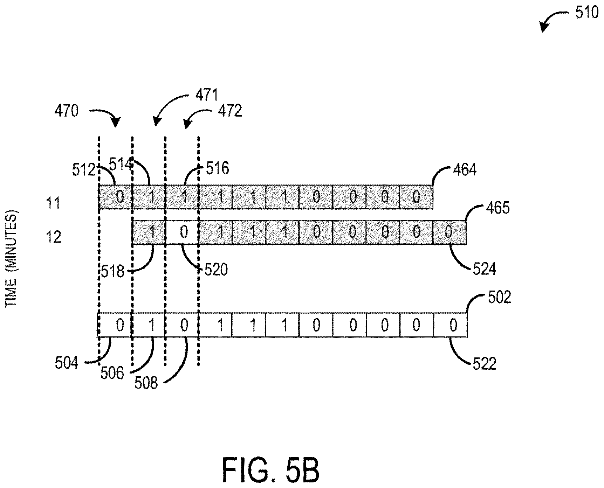

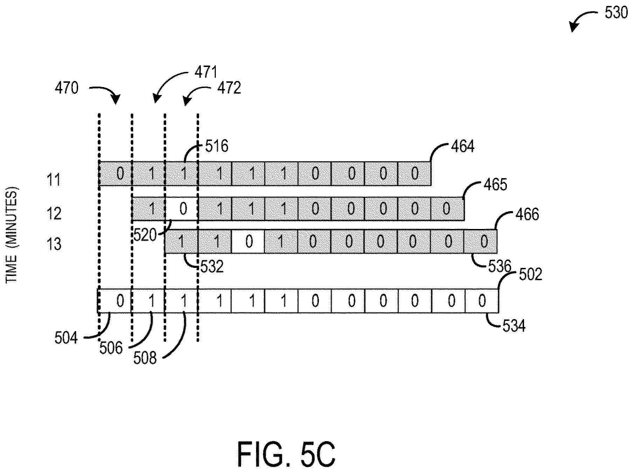

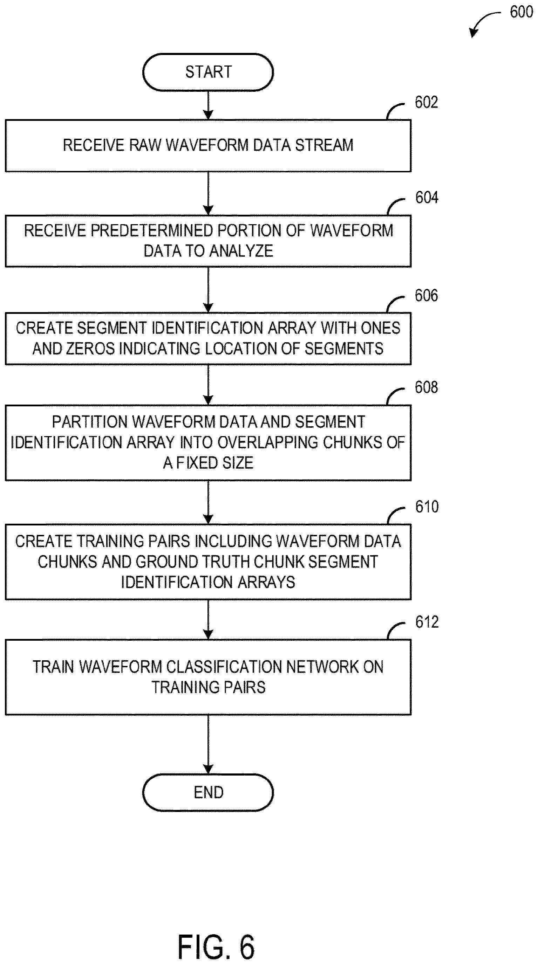

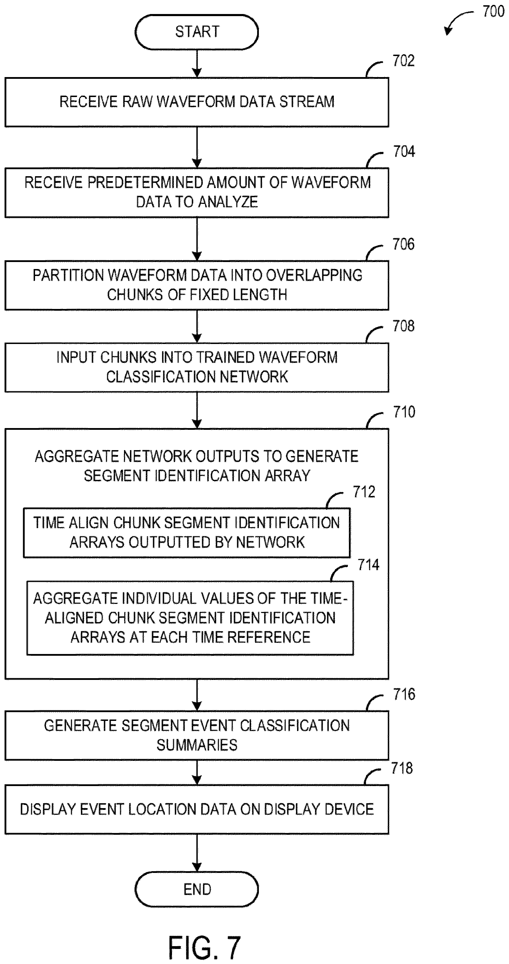

Methods and systems are provided herein for processing waveform data using a waveform processing system, such as the waveform processing system 102 of FIG. 1. The waveform processing system may process raw waveform data using a trained waveform classification neural network, as shown in FIG. 2B. The waveform classification neural network may be trained as described with respect to the exemplary neural network training system shown in FIG. 2A. The waveform processing system may take as input a stream of waveform data values, and may output a corresponding stream of codified values indicating the presence or absence of an event in the waveform data. The stream of codified values may include zeros (indicating the absence of the event) and ones (indicating the presence of the event), as shown in FIG. 3. The stream of waveform data may be divided into chunks to be inputted into the waveform classification neural network, as shown in FIG. 4A. Exemplary outputs of the waveform classification neural network for the chunks are shown in FIG. 4B. The outputs of the waveform classification neural network for the chunks may be aggregated as indicated in FIG. 4C, where individual steps of an aggregation procedure is shown in FIGS. 5A, 5B, and 5C. The waveform classification neural network may be trained by following one or more steps of the method shown in FIG. 6. A method for aggregating outputs of the waveform classification neural network to indicate locations of segments of raw waveform data including the specified events is shown in FIG. 7.

Referring to FIG. 1, a waveform processing system 102 of a clinical data management system 100 is shown, in accordance with an embodiment. In some embodiments, at least a portion of waveform processing system 102 is disposed at a device (e.g., edge device, server, etc.) communicably coupled to the clinical data management system 100 via wired and/or wireless connections. In some embodiments, at least a portion of waveform processing system 102 is disposed at a separate device (e.g., a workstation) which can receive waveform data from the clinical data management system 100 or from a storage device which stores the images/data generated by the clinical data management system 100.

Waveform processing system 102 includes a processor 104 configured to execute machine readable instructions stored in non-transitory memory 106. Processor 104 may be single core or multi-core, and the programs executed thereon may be configured for parallel or distributed processing. In some embodiments, the processor 104 may optionally include individual components that are distributed throughout two or more devices, which may be remotely located and/or configured for coordinated processing. In some embodiments, one or more aspects of the processor 104 may be virtualized and executed by remotely-accessible networked computing devices configured in a cloud computing configuration.

Non-transitory memory 106 may store a neural network module 108, a training module 110, an inference module 112, and waveform data 114. Neural network module 108 may include one or more AI models, such as a waveform classification network (e.g., neural network) for detecting the presence of predefined events in the waveform data. Neural network module 108 may include one or more trained and/or untrained neural networks and/or other AI models and may further include various data, or metadata pertaining to the one or more neural networks stored therein.

Training module 110 may comprise instructions for training one or more of the neural networks stored in neural network module 108, including the waveform classification network. In particular, training module 110 may include instructions that, when executed by the processor 104, cause waveform processing system 102 to conduct one or more of the steps of method 600 for training the waveform classification network, discussed in more detail below in reference to FIG. 6. In some embodiments, training module 110 includes instructions for implementing one or more gradient descent algorithms, applying one or more loss functions, and/or training routines, for use in adjusting parameters of the one or more neural networks of neural network module 108.

Inference module 112 may comprise instructions for implementing the waveform classification network to process waveform data 114, as described in greater detail below. In particular, inference module 112 may include instructions that, when executed by the processor 104, cause waveform processing system 102 to conduct one or more of the steps of method 700 for detecting the presence of the predefined events using the waveform classification network, discussed in more detail below in reference to FIG. 7.

Waveform data 114 may include waveform data acquired via a patient monitor 136. The waveform data may include, for example, fetal monitor data, electrocardiogram (ECG) data, electroencephalogram (EEG) data, intensive care unit (ICU) patient monitoring data, electromyography (EMG) data, and/or other types of waveform data. The waveform data may include a plurality of data values generated by patient monitor 136. The data values may be measured by patient monitor 136 at periodic intervals (e.g., once per second), and transmitted to one or more listening devices including waveform processing system 102. For example, in one embodiment patient monitor 136 is a heart rate monitor that monitors a heart rate of a patient in a hospital, and transmits the heart rate to waveform processing system 102 as a continuous stream of data. A caregiver of the patient may view the heart rate via a display device 134 of waveform processing system 102. For the purposes of this disclosure, the measured data values are described as being streamed wirelessly to waveform processing system 102. However, in other embodiments, the data values may be transmitted to waveform processing system 102 via a wired connection.

In some embodiments, the non-transitory memory 106 may include components disposed at two or more devices, which may be remotely located and/or configured for coordinated processing. In some embodiments, one or more aspects of the non-transitory memory 106 may include remotely-accessible networked storage devices configured in a cloud computing configuration.

Waveform processing system 102 may be operably/communicatively coupled to a user input device 132 and display device 134. User input device 132 may comprise one or more of a touchscreen, a keyboard, a mouse, a trackpad, a motion sensing camera, or other device configured to enable a user to interact with and manipulate data within waveform processing system 102. Display device 134 may include one or more display devices utilizing virtually any type of technology. In some embodiments, display device 134 may comprise a computer monitor, and may display waveform data received from patient monitor 136 and/or from waveform data 114. Display device 134 may be combined with processor 104, non-transitory memory 106, and/or user input device 132 in a shared enclosure, or may be peripheral display devices and may comprise a monitor, touchscreen, projector, or other display device known in the art, which may enable a user to view medical images produced by an medical imaging system, and/or interact with various data stored in non-transitory memory 106.

It should be understood that waveform processing system 102 shown in FIG. 1 is for illustration, not for limitation. Another appropriate waveform processing system may include more, fewer, or different components.

Referring to FIG. 2A, an exemplary waveform classification network training system 200 is shown, which may be used to train a waveform classification network 220. The waveform classification network 220 may be trained to identify segments of a waveform data stream 202 in which an event has occurred (e.g., a peak, trough, or aberration in the data). The waveform classification network 220 may be used by a waveform processing system, such as waveform processing system 102 of FIG. 1. Waveform classification network 220 may be stored within a neural network module 242 of the waveform processing system. Neural network module 242 may be a non-limiting example of neural network module 108 of waveform processing system 102 of FIG. 1.

The waveform data stream 202 may be generated by a patient monitoring device 201 (e.g., patient monitor 136 of FIG. 1). Waveform classification network training system 200 may include a waveform chunk generator 204, which may divide waveform data stream 202 into a plurality of overlapping waveform chunks 206, which may be stored in a training module 215, which may be a non-limiting example of training module 110 of waveform processing system 102 of FIG. 1. Each waveform chunk 206 may be a portion of waveform data stream 202. For example, waveform data stream 202 may be stored in a waveform data array in a buffer in a memory of the waveform processing system (e.g., non-transitory memory 106), and each waveform chunk 206 may be selected from the waveform data array based on an initial time reference. Dividing waveform data stream into the plurality of overlapping waveform chunks is described in greater detail below in relation to FIGS. 4A and 6.

Training module 215 may include a training data generator 210, which may generate a plurality of training pairs 212 from the waveform chunks 206. Each training pair may include a waveform chunk, and an indication of whether a segment or portion of a segment including the event is detected in the waveform chunk, as ground truth data. For example, the waveform chunk may include a first array of data values of waveform data stream 202, and the ground truth data may comprise a second, segment identification array of values of a same length as the first array, where the segment identification array includes a codification of a location of the segment or portion of the segment in the first array of data values. For example, the segment identification array may include ones and zeros, where a one indicates that a corresponding position of the first array of values includes a data value that is part of a segment, and a zero indicates that a corresponding position of the first array of values includes a data value that is not part of a segment. The codification of the segment identification array is described in greater detail in reference to FIG. 3.

A first portion of training pairs 212 may be assigned to a training dataset, a second portion of training pairs 212 may be assigned to a validation dataset, and a third portion of training pairs 212 may be assigned to a test dataset. In an embodiment, training pairs 212 may be assigned to either the training dataset, the validation dataset, or the test dataset randomly in a pre-established proportion. For example, training pairs 212 may be assigned to either the training dataset or the test dataset randomly such that 90% of the training pairs are assigned to the training dataset, and 10% of the training pairs are assigned to the test dataset and the validation dataset. It should be appreciated that the examples provided herein are for illustrative purposes, and the training pairs may be assigned to the training, validation, and test datasets via a different procedure and/or in a different proportion without departing from the scope of this disclosure.

Waveform classification network 220 may be trained on training pairs 212 to learn to identify segments where the event occurred. An example method for training the waveform classification network 220 is described in further detail below with respect to FIG. 6.

Waveform classification network training system 200 may include a validator 222 that validates the performance of the waveform classification network 220 using training pairs of the validation dataset and the test dataset. Validator 222 may use the validation dataset to prevent overfitting, and may output an assessment of a performance of the trained or partially trained waveform classification network 220 on the test dataset of training pairs. Training may be completed when waveform classification network 220 achieves a minimum error rate in detecting segments. After waveform classification network 220 is validated, waveform classification network 220 may be fully trained. Trained waveform classification network 230 (e.g., the validated waveform classification network 220) may be used to identify segments where a specified event has occurred in a new stream of waveform data. Trained waveform classification network 230 may be deployed in an inference module 240 of the waveform processing system (e.g., inference module 112 of FIG. 1).

Referring to FIG. 2B, a segment identification diagram 250 shows a series of processing steps performed by the waveform processing system to aggregate outputs of trained waveform classification network 230 of FIG. 2A, such that segments can be detected or identified in waveform data that spans a plurality of individual waveform chunks. As described in greater detail below, an accuracy of segment identification by the waveform processing system may be increased by aggregating the outputs as described herein.

A monitoring device 251 may generate a waveform data stream 252. Monitoring device 251 may be the same as monitoring device 201 of FIG. 2A, or monitoring device 251 may be a different monitoring device. Waveform chunk generator 204 may divide waveform data stream 252 into a set of overlapping chunks of waveform data that may be inputted into a trained waveform classification network 230, which may be trained in waveform classification network training system 200 of FIG. 2A. For each chunk inputted into trained waveform classification network 230 at regular intervals, trained waveform classification network 230 may output a chunk segment identification array 258 indicating locations of any segments in the chunk. Chunk segment identification arrays 258 may then be aggregated during an output aggregation process 260, to generate an overall segment identification array 262 for the waveform data. Segment identification array 262 may indicate locations of various instances of the pre-defined events in the waveform data stream 252. At periodic intervals, a segment event classification summary 264 may be generated, comprising a current state of segment identification array 262 at a time of generation. For example, a segment event classification summary 264 could be generated at each inference interval, or after a predetermined number of inference intervals. Segment event classification summaries 264 may be used by a segment display block 266 to display the locations of the events in waveform data stream 252 on a display device of the waveform processing system (e.g., display device 134). Aggregation of outputs of trained waveform classification network 230 into the segment identification array is described in greater detail below in FIGS. 4C-5C and 8.

In FIG. 3, a codification diagram 300 shows exemplary representations of waveform data and how a location of a segment including an event may be determined and indicated by a waveform processing system, such as waveform processing system 102 of FIG. 1. The waveform data may be a stream of data values generated by a streaming device such as a patient monitoring device (e.g., patient monitor 136 of FIG. 1 and/or monitoring device 201 of FIG. 2A). For example, in the depicted embodiment, the patient monitoring device may monitor a heart rate of a patient in a hospital. As such, the waveform data may be a continuous, ongoing stream of heart rate measurements. Alternatively, the waveform data may be a stored array of values corresponding to a duration of a previous stream of heart rate measurements. For example, the array may be stored in waveform data 114 of FIG. 1.

Codification diagram 300 includes a graphical display 320 of the waveform data, where the waveform data is indicated as a plot 302 on a set of coordinate axes. An x axis of graphical display 320 indicates time, and an exemplary variance in the waveform data (e.g., the heart rate) is shown on the y axis. 20 minutes of waveform data are shown in FIG. 3, which may comprise a predetermined amount of waveform data over which results are desired to be viewed. The predetermined amount of waveform data over which results are desired to be viewed may also be referred to herein as an aggregation interval, as described in greater detail below. The exemplary variance in the heart rate can also be seen in a waveform data array 330, where each value of waveform data array 330 corresponds to a heart rate depicted in graphical display 320. For example, a first point 314 on plot 302 corresponds to a heart rate of 77, shown in a position 322 of waveform data array 330. A second point 316 on plot 302 corresponds to a heart rate of 80, shown in a position 327 of waveform data array 330. Plot 302 and array 330 have a length of 20 minutes, where the values of plot 302 and array 330 may be selected from a stream of ongoing or stored waveform data. In other examples, the length may be greater or lesser.

Plot 302 indicates the occurrence of two events, where the events include an unexpected increase in heart rate. The events appear as peaks in plot 302. A first event 304 occurs centered at minute 4 of the waveform data, and a second event 306 occurs centered at minute 17 of the waveform data.

The waveform processing system may identify segments of the waveform data that include first event 304 and second event 306. Specifically, the waveform processing system may identify a first segment 310 including first event 304, and a second segment 312 including second event 306, where first segment 310 is bounded by a dashed line 350 and a dashed line 351, and second segment 312 bounded by a dashed line 352 and a dashed line 353.

Codification diagram 300 also includes a segment identification array 340, which may indicate a location of first segment 310 and second segment 312 within waveform data array 330. Segment identification array 340 may be generated as an output of the waveform processing system, as described in greater detail below. A length of segment identification array 340 may be equal to a length of waveform data array 330. The locations of first segment 310 and second segment 312 within waveform data array 330 may be indicated by a presence of either ones or zeros in segment identification array 340. In other words, a first value assigned to a position of segment identification array 340 may correspond to a second value included at the same position in waveform data array 330. The first value may be a one if the second value is included in a segment, and the first value may be a zero if the second value is not included in a segment.

For example, the heart rate corresponding to point 314 (e.g., 77 bpm) is included in waveform data array 330 at a fourth position of waveform data array 330 (e.g., corresponding to a measurement at a fourth minute of the waveform data). A one is assigned to the fourth position of segment identification array 340, indicating that the heart rate corresponding to point 314 is within a segment (e.g., segment 310). Additional heart rates within a threshold distance of point 314 are also included in the segment.

A first section 342 of segment identification array 340 includes ones, where first section 342 corresponds to first segment 310. First section 342 includes ones in a second position of segment identification array 340, a third position of segment identification array 340, a fourth position of segment identification array 340, a fifth position of segment identification array 340, and a sixth position of segment identification array 340, indicating that data values at the second position of waveform data array 330, the third position of waveform data array 330, the fourth position of waveform data array 330, the fifth position of waveform data array 330, and the sixth position of waveform data array 330 are included in first segment 310. Similarly, a second section 344 of segment identification array 340 includes ones, where second section 344 corresponds to second segment 312. Second section 344 includes ones in a 14^{th} position of segment identification array 340, a 15^{th} position of segment identification array 340, a 16^{th} position of segment identification array 340, a 17^{th} position of segment identification array 340, an 18^{th} position of segment identification array 340, and a 19^{th} position of segment identification array 340, indicating that data values at the 14^{th} position of waveform data array 330, the 15^{th} position of waveform data array 330, the 16^{th} position of waveform data array 330, the 17^{th} position of waveform data array 330, the 18^{th} position of waveform data array 330, and the 19^{th} position of waveform data array 330 are included in second segment 312. All other positions of segment identification array 340 include zeros, indicating that values at corresponding positions of waveform data array 330 are not included in any segments (e.g., that no events were detected in the corresponding positions).

Thus, the waveform processing system may process the data values of waveform data array 330 to determine locations of any events, and populate segment identification array 340 with ones or zeros to mark the locations. The waveform processing system may then use segment identification array 340 to inform a display of the locations of first segment 310 and second segment 312 within a representation of the waveform data on a display device of the waveform processing system. In various embodiments, processing the data values of waveform data array 330 to determine locations of any events may include inputting the data values into an AI model such as a neural network.

FIG. 4A shows a chunking diagram 400 indicating how the data values may be processed by the AI model in chunks. Chunking diagram 400 shows time along an x-axis corresponding to waveform data array 330 of FIG. 3 (e.g., the predetermined amount of waveform data over which results are desired to be viewed), and a set of chunks into which the values of waveform data array 330 are assigned. Chunking diagram 400 shows waveform data array 330 being divided into 11 chunks 401, where each chunk includes a predefined number of data values of waveform data array 330. In FIG. 4C, the predefined number is 10. In other embodiments, the predefined number may be a different number. With a chunk size of 10, 20 minutes of waveform data may be processed by exactly 11 overlapping chunks.

Chunks 1 to 11 may be offset from each other by a predefined inference interval. In FIG. 4A, the inference interval is one minute. At the passage of each inference interval (e.g., every minute), a new chunk may be inputted into the AI model for processing. Thus, processing the chunks may include using the AI model to perform sliding window inferences on the waveform data.

At a first inference interval, a first data value (e.g., heart rate) of waveform data array 330 may be assigned to a first position of a first chunk 402. At a second inference interval, a second data value of waveform data array 330 may be assigned to a second position of first chunk 402. Additionally, the second data value may be assigned to a first position of a second chunk 404. At a third inference interval, a third data value of waveform data array 330 may be assigned to a third position of first chunk 402, a second position of second chunk 404, and a first position of a third chunk 406, and so on. In this way, a plurality of overlapping chunks of waveform data may be created from waveform data array 330, where each overlapping chunk is offset from a previous overlapping chunk and a subsequent overlapping chunk by the inference interval. After 11 chunks, all of the data values of waveform data array 330 may be represented in chunks, where a last data value of an 11^{th} chunk may be the last data value of waveform data array 330.

When the predefined number of data values of an overlapping chunk has been obtained, meaning that a data value is included in each position of the relevant chunk, the relevant chunk may be inputted into the AI model. A first dashed line 410 shows a completion of first chunk 402. A second dashed line 412 shows a completion of second chunk 404, and a third dashed line 414 shows a completion of third chunk 406. Thus, after 10 inference intervals have passed (e.g., at minute 10), first chunk 402 may include the predefined number of data values, and may be inputted into the AI model. After the passage of a subsequent inference interval, at minute 11, second chunk 404 may include the predefined number of data values, and may be inputted into the AI model. After the passage of a subsequent inference interval, at minute 12, third chunk 406 may include the predefined number of data values, it may be inputted into the AI model. In this way, data values of waveform data array 330 are assigned to chunks at each inference interval, and after a minimum number of data values for processing (e.g., the predefined number) have been collected in a chunk, the chunk is submitted to the AI model. (Note that this implies that results may be displayed with a 10 second delay).

FIG. 4B shows a sliding window inferencing diagram 430, where sliding window inferencing diagram 430 shows a set of exemplary outputs 434 of the AI model corresponding to a set of exemplary inputs 432 inputted into the AI model in chunks as described above in reference to FIG. 4A. Sliding window inferencing diagram 430 shows a timing with which chunks 1 to 11 are inputted into the AI model, based on the same timeline shown in FIG. 4A. As in FIG. 4A, first chunk 402 starts at the first inference interval and ends at minute 10; second chunk 404 starts at the second inference interval and ends at minute 11; and third chunk 406 starts at the third inference interval and ends at minute 12. At minute 11, a first array of data values of first chunk 402 is inputted into the AI model, and the AI model outputs a corresponding first chunk segment identification array 436 of a same length as the first array. First chunk segment identification array 436 includes ones and zeros, where the ones indicate a location of a segment within first chunk 402. For example, a zero is included at a first position 443 of first chunk segment identification array 436, indicating that a first data value 446 (e.g., 72) of first chunk 402 is not included in a segment. A one is included at a second position 444 of first chunk segment identification array 436, indicating that a second data value 448 (e.g., 73) of first chunk 402 is included in a segment. First chunk segment identification array 436 includes a span 442 of consecutive ones indicating a location of a segment 431 in first chunk 402 (corresponding to first section 342 of FIG. 3). Specifically, ones are included in a second position (444), a third position, a fourth position, a fifth position, and a sixth position of first chunk segment identification array 436, indicating the presence of segment 431 at a second position, a third position, a fourth position, a fifth position, and a sixth position of first chunk 402, also indicated by bold text in FIG. 4B.

When second chunk 404 is inputted into the AI model at minute 12, the AI model outputs a second chunk segment identification array 438, where second chunk segment identification array 438 includes ones indicating the location of segment 431 in second chunk 404. As such, the first five positions of second chunk segment identification array 438 include ones, indicating the presence of segment 431 at the first five positions of second chunk 404.

When third chunk 406 is inputted into the AI model at minute 13, the AI model outputs a third chunk segment identification array 440, where third chunk segment identification array 440 includes ones indicating the location of segment 431 in third chunk 406. The first four positions of third chunk segment identification array 440 include ones, indicating the presence of a portion of segment 431 at the first four positions of third chunk 406.

Because each chunk inputted into the AI model starts at a subsequent inference interval, the values included the positions of each chunk segment identification array outputted by the AI model "slide" vertically up in sliding window inferencing diagram 430, where at each successive inference interval, a one or a zero at a first position of a relevant chunk segment identification array is eliminated, and a one or a zero is appended at a last position of the relevant chunk segment identification array. In this way, the sliding window inferencing is performed over a series of chunks, where consecutive outputs generated by the AI model at consecutive inference intervals are used to infer the presence of segments in each chunk of the series of chunks. The chunk segment identification arrays outputted by the AI model may then be aggregated to generate a composite segment identification array (e.g., segment identification array 340 of FIG. 3) that may indicate the locations of one or more segments found in a corresponding stream of waveform data (e.g., waveform data array 330).

Aggregating the chunk segment identification arrays outputted by the AI model may include time-aligning the chunk segment identification arrays, and aggregating values (e.g., ones or zeros) occurring in a plurality of chunks at a same time reference. For example, a time reference 450 may be at the third inference interval, indicated by dashed lines 451 and 452. At time reference 450, a single data value (e.g., 75) is recorded by the waveform processing system. The single data value is included in first chunk 402 in the third position; in second chunk 404 in the second position; and in third chunk 406 in the first position (e.g., all at the same time at minute 3). However, since first chunk 402, second chunk 404, and third chunk 406 are inputted into the AI model at different times, corresponding output values associated with the single data value may be included in first chunk segment identification array 436, second chunk segment identification array 438, and third chunk segment identification array 440 at different times. Therefore, during aggregation, first chunk segment identification array 436, second chunk segment identification array 438, and third chunk segment identification array 440 may be time-aligned (e.g., offset by one inference interval each) to ensure that the output values corresponding to time reference 450 are aggregated, and not aggregated with output values corresponding to different time references.

A set of time-aligned outputs of first, second, and third chunk segment identification arrays 436, 438, and 440, respectively, is indicated in sliding window inferencing diagram 430 by a dashed line 454. Dashed line 454 shows that a first output value of 1 located in a third position 455 of first chunk segment identification array 436 and associated with a third position of first chunk 402, is time-aligned with a second output value of 1 located in a second position 456 of second chunk segment identification array 438 and associated with a second position of second chunk 404, and a third output value of 1 located in a first position 457 of third chunk segment identification array 440 and associated with a first position of third chunk 406. During aggregation, the first output value, the second output value, and the third output value may be aggregated to generate an aggregated value, to be assigned to the (global) segment identification array for the waveform data. For example, the first output value, the second output value, and the third output value may be averaged to generate the aggregated value.

In the depicted example, the first output value, the second output value, and the third output value are equal, indicating that the AI model outputted a same result for each of first chunk 402, second chunk 404, and third chunk 406. However, the AI model may not always correctly identify a presence of a segment in each chunk including the segment, where time-aligned output values of a plurality of chunks at a given time reference may not all be the same. By aggregating the time-aligned output values over the plurality of chunks, an accuracy of an overall output of the waveform processing system may be increased.

FIG. 4C shows an aggregation diagram 460 illustrating an exemplary aggregation of a plurality of chunk segment identification arrays 462, such as first, second, and third chunk segment identification arrays 436, 438, and 440, respectively. Aggregation diagram 460 shows an aggregation of data values outputted by the AI model for the 11 chunks used to process waveform data array 330. The data values outputted by the AI model for the 11 chunks are aggregated into segment identification array 340 of FIG. 3. A time at which the AI model outputs each chunk segment identification array of the plurality of chunk segment identification arrays 462 is shown along a left side of aggregation diagram 460, where a first chunk segment identification array 464 outputted by the AI model corresponds to first chunk segment identification array 436 of FIG. 4B; a second chunk segment identification array 465 outputted by the AI model corresponds to second chunk segment identification array 438 of FIG. 4B; and a third chunk segment identification array 466 outputted by the AI model corresponds to third chunk segment identification array 440 of FIG. 4B. First chunk segment identification array 464 is outputted by the AI model at minute 11; second chunk segment identification array 465 is outputted by the AI model at minute 12; third chunk segment identification array 466 is outputted by the AI model at minute 13; and so on.

Chunk segment identification arrays 462 are time-aligned, such that data values included in each chunk segment identification array from a same time reference are vertically aligned in columns. In other words, chunk segment identification arrays 462 are each offset by the inference interval (e.g., one minute). Thus, each aggregate data value of segment identification array 340 is a result of aggregating time-aligned output values of one or more chunk segment identification arrays vertically aligned above it. For example, a first value 480 of segment identification array 340 is an aggregation of a first value of first chunk segment identification array 464 at time reference 470; a second value 481 of segment identification array 340 is an aggregation of a second value of first chunk segment identification array 464 and a first value of second chunk segment identification array 465 at time reference 471; a third value 482 of segment identification array 340 is an aggregation of a third value of first chunk segment identification array 464, a second value of second chunk segment identification array 465, and a first value of third chunk segment identification array 466 at time reference 472; and so on.

Because each chunk segment identification array is offset from a previous chunk segment identification array by the inference interval, a number of data values aggregated to generate an aggregate output value of segment identification array 340 may vary. In other words, first value 480 of segment identification array 340 may be generated by aggregating output one value of a single chunk segment identification array; second value 481 of segment identification array 340 may be generated by aggregating output values of two chunk segment identification arrays; third value 482 of segment identification array 340 may be generated by aggregating output values of three chunk segment identification arrays; and so on. Thus, the number of output values aggregated to generate an aggregate output value of segment identification array 340 may initially increase from one, to a maximum number of output values at a middle of segment identification array 340, and then may decrease back down to one when a last value 484 of segment identification array 340 may be generated by aggregating a last output value of a single chunk segment identification array at a time reference 474. The maximum number of output values may be aggregated at a time reference 473, in the middle of segment identification array 340. In the depicted example, the maximum number of output values is 10.

An advantage of aggregating AI model outputs over a plurality of overlapping chunks is that errors (e.g., misclassifications) made by the AI model may be corrected. The AI model may periodically misclassify a data value of waveform data array 330 as being included in a segment, or not being in a segment. In FIG. 4C, periodic misclassifications of the AI model are shown with a white background, and correct classifications of the AI model are shown with a gray background. For example, second chunk segment identification array 465 includes a zero in a second position, when a correct value would be a one. Similarly, chunk segment identification array 466 includes a zero in a third position, when a correct value would be a one. By generating an aggregate value from various instances of output values at a given time reference, if a majority of the output values are correct classifications, the aggregate value may be a correct classification. Put another way, for the aggregate value to be a misclassification, the majority of the output values of the chunk segment identification arrays that are aggregated have to be misclassifications.

Various aggregation strategies may be used to aggregate the output values of the chunk segment identification arrays. In some embodiments, the output values may be averaged to generate an average output value. If the average output value is greater than a threshold value (e.g., .5), a one may be assigned to segment identification array 340. If the average output value is less than or equal to the threshold value, a zero may be assigned to segment identification array 340. In other words, the average output value may be rounded up or down to generate the aggregate output value.

For example, at time reference 472, three values are aggregated to produce third value 482 of segment identification array 340: a third value of first chunk segment identification array 464, which is a correct classification indicated by a one; a second value of second chunk segment identification array 465, which is an incorrect classification indicated by a zero; and a first value of third chunk segment identification array 466, which is a correct classification indicated by a one. An average of the three values is .666, which may be rounded up to 1 for third value 482. Alternatively, if two of the three values were zeros, the average of the three values would be .333, which may be rounded down to generate a zero for third value 482.

As another example, at time reference 473, ten output values from ten chunk segment identification arrays are aggregated to generate an aggregate 10^{th} value 483 of segment identification array 340. At time reference 473, eight of the ten output values are zeros, (correctly) classifying a 10^{th} value of waveform data array 330 as not being included in a segment. Two of the ten output values are ones, indicating misclassifications of the AI model. An average of the ten output values is .2, which may be rounded down to generate a 0 at aggregate 10^{th} value 483 of segment identification array 340. In this way, the misclassifications of the AI model are ignored, resulting in a more accurate identification of segments in waveform data array 330.

In some embodiments, the output values of the chunk segment identification arrays may not be zeros or ones. For example, the AI model may be trained using zeros and ones, and may output values within a range from zero to one. In some embodiments, a threshold may be applied to the output values to round the output values up or down to generate ones or zeros in the chunk segment identification arrays. In other embodiments, the output values may not be rounded up or down to generate ones or zeros, and the chunk segment identification arrays may include the output values of the AI model within the range from zero to one. In such cases, the aggregate output value may be more accurate.

Another aggregation strategy could be to use a weighted average rather than an average. For example, when generating an aggregated value for a given position, more weight could be assigned to values where the given position occurs in the middle of the chunk, and less weight could be assigned to values where the given position occurs at the end or beginning of a chunk.

As an example of how the aggregation may be performed over various time instances, FIGS. 5A, 5B, and 5C show consecutive steps in an aggregation procedure.

FIG. 5A shows a first aggregation step 500 at a first time instance at which an output of the AI model is received, where the first time instance occurs at an 11^{th} minute of processing performed by the waveform processing system. At the first time instance, first chunk segment identification array 464 is generated as a first output of the AI model (e.g., after a 10 minute delay). In one embodiment, at the first time instance, an initial segment identification array 502 is created, where initial segment identification array 502 may be a first part of segment identification array 340.

FIG. 5B shows a second aggregation step 510 at a second time instance at which an output of the AI model is received, where the second time instance occurs at a 12^{th} minute of processing performed by the waveform processing system. At the second time instance, second chunk segment identification array 465 is generated as a second output of the AI model. At the second time instance, first chunk segment identification array 464 and second chunk segment identification array 465 are aggregated. During aggregation, first chunk segment identification array 464 and second chunk segment identification array 465 are time-aligned, such that a first set of values of first chunk segment identification array 464 and a second set of values of second chunk segment identification array 465 may be compared with respect to a series of time references. At each time reference of the series of time references, corresponding values of first chunk segment identification array 464 and second chunk segment identification array 465 are aggregated, and an aggregate output value is included or updated in segment identification array 502.

For example, at first time reference 470 (e.g., of FIG. 4C), a single output value at a first position 512 of first chunk segment identification array 464 is used as a first aggregate value 504 of segment identification array 502. At second time reference 471, an output value at a second position 514 of first chunk segment identification array 464 and an output value at a first position 518 of second chunk segment identification array 465 are aggregated to generate a second aggregate value 506. At third time reference 472, an output value at a third position 516 of first chunk segment identification array 464, and an output value at a second position 520 of second chunk segment identification array 465 are aggregated to generate a third aggregate value 508. Because the output value at third position 516 of first chunk segment identification array 464 is a one, and the output value at second position 520 of second chunk segment identification array 465 is a zero, when the output values are averaged, third aggregate value 508 may be changed from a 1 (generated at the first step shown in FIG. 5A) to a zero. Additionally, an aggregate output 522 is appended to segment identification array 502, based on a last output value 524 of second chunk segment identification array 465.

FIG. 5C shows a third aggregation step 530 at a third time instance at which an output of the AI model is received, where the third time instance occurs at a 13^{th} minute of processing performed by the waveform processing system. At the third time instance, third chunk segment identification array 466 is generated as a third output of the AI model. At the third time instance, first chunk segment identification array 464, second chunk segment identification array 465, and third chunk segment identification array 466 are aggregated. During aggregation, first chunk segment identification array 464, second chunk segment identification array 465, and third chunk segment identification array 466 are time-aligned, such that a first set of values of first chunk segment identification array 464, a second set of values of second chunk segment identification array 465, and a third set of values of third chunk segment identification array 466 may be compared with respect to a series of time references. At each time reference of the series of time references, corresponding values of first chunk segment identification array 464, second chunk segment identification array 465, and third chunk segment identification array 466 are aggregated, and an aggregate output value is included or updated in segment identification array 502.

At third time reference 472, first aggregate value 504 and second aggregate value 506 of segment identification array 502 are retained from FIG. 5B. An output value at a third position 516 of first chunk segment identification array 464, an output value at a second position 520 of second chunk segment identification array 465, and an output value at a first position 532 of third chunk segment identification array 466 are aggregated to regenerate third aggregate value 508. Because an average of the output value at first position 532 of third chunk segment identification array 466 (e.g., a one), the output value at second position 520 of second chunk segment identification array 465 (e.g., a zero), and the output value at third position 516 of first chunk segment identification array 464 (e.g., a one) is .666, third aggregate value 508 may be changed back from a 0 (generated at the second step shown in FIG. 5B) to a one. In this way, the addition of data from third chunk segment identification array 466 corrects the misclassification made by the AI model at time reference 471 shown in second chunk segment identification array 465. Additionally, an aggregate output 534 is appended to segment identification array 502, based on a last output value 536 of third chunk segment identification array 466.

Thus, as additional output data from additional chunk segment identification arrays is aggregated, output values are added at an end of segment identification array 502, and confirmed or corrected at a beginning of segment identification array 502. When a final chunk segment identification array outputted by the AI model for a last chunk of input data of waveform data array 330 (e.g., chunk 11 of FIG. 4A), a last output value of the last chunk may be appended to segment identification array 502, at which point segment identification array 502 may be identical to segment identification array 340 of FIG. 4C. As a result of the aggregating of output data from overlapping chunks of input data, a number of the individual output values aggregated to generate the segment identification array may increase with each time instance from one individual output value at a beginning of the segment identification array, to a maximum number of individual output values at a middle of the segment identification array, and may then decrease with each time instance from the maximum number of individual output values to one individual output value at an end of the segment identification array.

In another embodiment, the output data may not be aggregated and/or updated incrementally as described above, and the output data may be collected and aggregated to produce segment identification array 340 after the AI model has processed the plurality of chunks covering the predetermined amount of waveform data.

Referring now to FIG. 6, a flowchart is shown of a method 600 for training a waveform classification network, such as waveform classification network 220 of FIG. 2A, according to an exemplary embodiment. Waveform classification network 220 may be trained in accordance with the waveform classification network training system 200. In some embodiments, the waveform classification network may be a deep neural network with a plurality of hidden layers. For example, the waveform classification network may be a convolutional neural network (CNN). Method 600 and the other methods described herein may be executed by a processor of a waveform processing system, such as waveform processing system 102 of FIG. 1. Operations of method 600 may be stored in non-transitory memory of the waveform processing system (e.g., in a training module such as the training module 110 of the waveform processing system 102 of FIG. 1) and executed by a processor of the waveform processing system (e.g., the processor 104 of waveform processing system 102 of FIG. 1). The waveform classification network may be trained on training data generated as described in reference to FIG. 2A and FIGS. 4A-4C. In some embodiments, the training data may comprise waveform data stored in an waveform dataset of the waveform processing system, such as waveform data 114 of waveform processing system 102 of FIG. 1.

Method 600 begins at 602, where method 600 includes receiving a stream of raw waveform data including a plurality of instances of a specified event, such as a peak, trough, or aberration in the waveform data. In various embodiments, the raw waveform data stream may be generated by a source such as a patient monitor positioned on a patient of a healthcare system. For example, the patient monitor may be a fetal monitor that monitors a fetus of a pregnant woman, or a different kind of patient monitor. In other embodiments, the raw waveform data stream may be generated by a different device other than a patient monitor.

At 604, method 600 includes receiving a predetermined portion (e.g., an amount) of the waveform data to analyze. For training purposes, the predetermined amount of the waveform data to analyze may vary. The predetermined amount of waveform data may be a portion where a desired amount of events are included in the waveform data. For example, the predetermined amount of the waveform data to analyze may include three events, or 10 events, or a greater or lesser number of events. The predetermined amount of the waveform data may be selected based on a quality of the waveform data. In some examples, method 600 may be performed various times to train the waveform classification network, each time using different predetermined portions and/or amounts of waveform data. The predetermined amount of the waveform data may be stored in a waveform data array (e.g., waveform data array 330 of FIG. 3), where the waveform data array includes data values collected a regular intervals from the source.

At 606, method 600 includes creating a segment identification array (e.g., segment identification array 340) including ones and zeros indicating locations of the specified events in the waveform data, as described above. The segment identification array may have a length that is equal to a length of the waveform data array, such that values of the segment identification array have a 1:1 correspondence with data values of the waveform data array. Each value of the segment identification array indicates whether the corresponding data value of the waveform data array is included in a segment, where a one indicates that the corresponding data value is included in the segment, and a zero indicates that the corresponding data value is not included in the segment. In various embodiments, the ones and zeros may be assigned to positions of the segment identification array manually.

At 608, method 600 includes partitioning data values of the waveform data array into a plurality of overlapping chunks (e.g., waveform chunks 206 of FIG. 2B) of a predefined length or size. For example, the predefined length may be 10 data values. The waveform chunks may be created as described above in reference to FIG. 4A. Similarly, the segment identification array may be partitioned into a respective plurality of chunk segment identification arrays (e.g., chunk segment identification arrays 462 of FIG. 4C), where each chunk segment identification array maintains a 1:1 correspondence with a relevant overlapping chunk.

For example, a first chunk may be created from the waveform data array including ten data values starting with a first data value and ending with a 10^{th} data value of the waveform data array. A corresponding first chunk segment identification array may be created from the segment identification array including ten values starting with a first value and ending with a 10^{th} value of the segment identification array, where the values of the corresponding first chunk segment identification array have a 1:1 correspondence with the data values of the first chunk. A second chunk may be created from the waveform data array including ten data values starting with a second data value and ending with an 11^{th} data value of the waveform data array, such that the second chunk is offset from the first chunk by one data value, and overlaps the first chunk when plotted on a timeline. A corresponding second chunk segment identification array may be created from the segment identification array including ten values starting with a second value and ending with an 11^{th} value of the segment identification array, where the values of the corresponding second chunk segment identification array have a 1:1 correspondence with the data values of the second chunk, and so on.

At 610, method 600 includes creating a plurality of training pairs, where each training pair of the plurality of training pairs includes an array of data values corresponding to a chunk, as input data, and a corresponding chunk segment identification array indicating (e.g., with ones and zeros) the locations of any segments included in the chunk. Once the training pairs have been created, the training pairs may be divided into a training dataset, a validation dataset, and a test dataset, as described above in reference to FIG. 2A.

At 612, method 600 includes training the waveform classification network on the training pairs. During training, the waveform classification network may be configured to iteratively adjust one or more of a plurality of weights of the waveform classification network in order to minimize a loss function, based on a difference between an output of the waveform classification network for a given waveform chunk and the corresponding target, ground truth chunk segment identification array assigned to the waveform chunk in the relevant training pair. In one embodiment, the loss function is a Mean Absolute Error (MAE) loss function. It should be appreciated that the examples provided herein are for illustrative purposes, and other types of loss functions may be used without departing from the scope of this disclosure.

The difference (or loss), as determined by the loss function, may be back-propagated through the waveform classification network to update the weights (and biases) of the hidden layers. In some embodiments, back propagation of the loss may occur in accordance with a gradient descent algorithm, wherein a gradient of the loss function (a first derivative, or approximation of the first derivative) is determined for each weight and bias of the waveform classification network. Each weight (and bias) of the waveform classification network is then updated by adding the negative of the product of the gradient determined (or approximated) for the weight (or bias) with a predetermined step size. Updating of the weights and biases may be repeated until the weights and biases of the waveform classification network converge, or the rate of change of the weights and/or biases of the waveform classification network for each iteration of weight adjustment are under a threshold. Method 600 ends.

Referring now to FIG. 7, a flowchart is shown of a method 700 for deploying a trained waveform classification network, such as trained waveform classification network 230 of FIG. 2B, to perform inferences on waveform data as part of a waveform data processing system (e.g., waveform processing system 102 of FIG. 1). The inferences may be used to determine a location of one or more predefined events in a stream of waveform data, such as a peak, trough, or aberration. In an embodiment, operations of method 700 may be stored in non-transitory memory of the waveform processing system (e.g., in an inference module such as inference module 112) and executed by a processor of the waveform processing system (e.g., processor 104).

Method 700 begins at 702, where method 700 includes receiving a stream of new waveform data. In various embodiments, the waveform data stream may be generated by a patient monitor positioned on a patient of a healthcare system, as described above. The waveform data stream may not be generated by the same patient monitor used to generate the training data. In other words, a first patient monitor may be used to generate a first raw waveform data stream used to train the waveform classification network, and a second patient monitor may be used to generate a second raw waveform data stream received at 702. In other embodiments, the waveform data stream may be generated by a different device other than a patient monitor.

At 704, method 700 includes receiving a predetermined amount of waveform data to analyze. For example, from the (ongoing) stream of raw waveform data, a caregiver of the patient and user of the waveform processing system may be interested in a particular portion corresponding to a particular time period. For example, the caregiver may wish to analyze 20 minutes of waveform data, or an hour of waveform data, or a different amount of waveform data of the stream of raw waveform data. The predetermined amount of waveform data may be referred to as an aggregation interval, meaning, an amount of data over which overlapping analyses of the waveform classification network may be aggregated to generate a set of aggregate results.

At 706, method 700 includes dividing the waveform data into a plurality of overlapping chunks of a predefined fixed length, as described above. The predefined fixed length may be the same as the predefined fixed length used during training of the waveform classification network. In various embodiments, the predefined fixed length may be ten, where each chunk of the overlapping chunks includes ten data values.

At 708, method 700 inputting each chunk of the plurality of overlapping chunks into the trained waveform classification network. The chunks may be inputted into the trained waveform classification network at predefined inference intervals, where a predefined inference interval may be a desired amount of time between outputs of the trained waveform classification network. For example, at a first inference interval, a first chunk may be inputted into the trained waveform classification network and a first output (e.g., a chunk segment identification array) may be generated; at a second inference interval, a second chunk may be inputted into the trained waveform classification network and a second output may be generated; at a third inference interval, a third chunk may be inputted into the trained waveform classification network and a third output may be generated; and so on. The predefined inference interval may also correspond to an amount of time that each chunk of the overlapping chunks is offset from a preceding chunk. In other words, the predefined inference interval may be one minute, where each chunk of the overlapping chunks begins one minute after a preceding chunk, and a new output of the trained waveform classification network is received every minute by the waveform processing system.

At 710, method 700 includes aggregating the outputs of the trained waveform classification network received at each inference interval, for each chunk inputted into the trained waveform classification network at the inference interval, to generate an overall segment identification array for the waveform data. The overall segment identification array may have a length that is greater than the length of one or more chunks.

At 712, aggregating the outputs of the trained waveform classification network received at each inference interval includes time-aligning a plurality of chunk segment identification arrays based on the inference interval, as described above in reference to FIG. 4C. When the plurality of chunk segment identification arrays are time-aligned, individual output values of each time-aligned chunk segment identification array that were recorded by the waveform processing system at a same time reference may be directly compared.

At 714, aggregating the outputs of the trained waveform classification network received at each inference interval includes aggregating the individual output values of each time-aligned chunk segment identification array referring to a same time reference, to generate aggregate output values for each time reference. The aggregate output values may be stored in a segment identification array. The segment identification array may increase in length during processing until the predefined amount of the waveform data has been obtained. In various embodiments, aggregating the individual output values may include averaging the individual output values, and applying a threshold to assign either a zero or a one to a relevant position of the segment identification array.

At 716, method 700 includes generating segment event classification summaries for a user of the waveform processing system. As the trained waveform classification network outputs are aggregated, segment event classification summaries are periodically generated that indicate locations of any predefined events in the waveform data up to a time of the most recent segment event classification summary. The segment event classification summaries may include a most recently updated segment identification array for the waveform data received up to the time, minus a delay corresponding to the length of each chunk (e.g., a time used to generate a minimum amount of data to input into the trained waveform classification network). When the waveform processing system receives a segment event classification summary, the waveform processing system may use the most recently updated segment identification array to display the locations of any events detected on the display concurrently with the waveform data.

At 718, method 700 includes using the segment event classification summaries to generate a visual display of locations of any segments detected in the waveform data up to a time of a relevant segment event classification summary. In other words, as the waveform data is processed by the waveform processing system, the waveform data may be displayed on a display of the waveform processing system (e.g., display device 134) in real time. For example, the waveform data may be plotted over time in a continuously updating fashion. A caregiver of the patient may be using the waveform processing system to monitor the patient. When a segment event classification summary is generated, the most recently updated segment identification array included in the segment event classification summary may be used to inform a display of segments detected in the waveform data.

For example, the waveform processing system may iterate through each value of the most recently updated segment identification array until a one is detected. A time reference of the one (e.g., a position of the one in the most recently updated segment identification array) may be determined by the waveform processing system. The waveform processing system may use the time reference to identify a data value of the waveform data corresponding to the one. The data value of the waveform data corresponding to the one may be a first data value in a segment including the predefined event, whereby the waveform processing system may indicate the beginning of the segment on the waveform data. For example, a portion of the waveform data corresponding to the segment may be highlighted.

The waveform processing system may continue iterating through each value of the most recently updated segment identification array until a zero is detected. A time reference of the zero (e.g., a position of the zero in the most recently updated segment identification array) may be determined by the waveform processing system. The waveform processing system may use the time reference to identify a data value of the waveform data corresponding to the zero. The data value of the waveform data corresponding to the zero may be a last data value in the segment including the predefined event, whereby the waveform processing system may indicate the end of the segment on the waveform data. For example, the waveform data after the end of the segment may not be highlighted. In this way, the waveform processing system may continuously generate new chunks of waveform data, input the chunks into the trained waveform classification network, receive chunk segment identification arrays outputted by the trained waveform classification network, aggregate the chunk segment identification arrays as they are received to generate the segment event classification summaries, and update a display of the waveform data for the user in real time based on the segment event classification summaries.

Thus, a framework is described for analyzing waveform data of a longer duration than may be inputted into an AI model at one time, using sliding window inferencing. Analyzing the waveform data may include detecting aberrations or unexpected deviations in the waveform data. The waveform data may be inputted into the AI model and analyzed in chunks, and outputs of the AI model for each chunk may be aggregated in accordance with the methods described herein to generate results for the longer duration waveform data. Specifically, the chunks may be offset and overlapping, such that the outputs may be time-aligned to compare individual output values over a series of time references, where the time references correspond to times at which data values of the waveform data were collected by a source of the waveform data. Model output values occurring at each time reference across a plurality of chunks may be aggregated, to generate a set of aggregate output values that spans a predetermined amount of the waveform data, where the predetermined amount is greater than the size of a chunk (e.g., a desired amount of data for inputting into the AI model at one time). The outputs may be used to indicate the locations of detected segments in the longer duration waveform data on a display device of a waveform processing system.

In other words, the waveform data is collected and analyzed over a plurality of time windows, while the time windows move across the waveform data with each inference interval. As the time windows slide across the waveform data, the AI model can analyze relationships of individual data values with differing groups of neighboring data. By aggregating the outputs over a plurality of overlapping time windows, individual output values that are expected to match may be compared, and occasional misclassifications of the AI model may be corrected, leading to an increased accuracy of the AI model in detecting events in the waveform data. In this way, the waveform processing system may generate more precise and reliable event detection, which in a clinical setting may lead to better diagnostic results and/or patient outcomes.

The technical result of aggregating results of an AI model over a series of overlapping chunks of input data that are offset by an inference interval is that longer duration waveform data may be analyzed with a higher degree of accuracy than may be achieved using other aggregation strategies.

The disclosure also provides support for a method for a waveform processing system, the method comprising: displaying, on a display device of the waveform processing system, a location in a stream of waveform data of a segment including a specified event, wherein the location of the segment is identified based on aggregating outputs of an al model performing sliding window inferences on the stream of waveform data. In a first example of the method, the specified event is an aberration or variation in a plot of waveform data from an expected plot. In a second example of the method, optionally including the first example, performing the sliding window inferences on the stream of waveform data further comprises: dividing the waveform data into a plurality of overlapping chunks, each overlapping chunk comprising an array of consecutive data values extracted from the stream of waveform data over a specified duration, the array having a predefined size, at a predefined inference interval, inputting the array of consecutive data values of a chunk of the overlapping chunks of waveform data into the AI model, and receiving an output of the AI model, the output comprising a respective chunk segment identification array of output values of the predefined size, the output values indicating the location of the segment in the chunk of the waveform data. In a third example of the method, optionally including one or both of the first and second examples, a start of each overlapping chunk of the plurality of overlapping chunks is offset from a start of a preceding overlapping chunk by the predefined inference interval. In a fourth example of the method, optionally including one or more or each of the first through third examples, the respective chunk segment identification array includes a plurality of ones and zeros, where a one in a position of the chunk segment identification array indicates that a data value in a corresponding position of the inputted array of consecutive data values is included in the segment, and a zero in a position of the chunk segment identification array indicates that a data value in a corresponding position of the array of consecutive data values is not included in the segment. In a fifth example of the method, optionally including one or more or each of the first through fourth examples, the specified duration of each overlapping chunk is longer than the predefined inference interval. In a sixth example of the method, optionally including one or more or each of the first through fifth examples, aggregating the outputs of the AI model further comprises: for a predefined portion of the waveform data: time-aligning a plurality of respective chunk segment identification arrays outputted by the AI model corresponding to the predefined portion, aggregating individual output values of the time-aligned chunk segment identification arrays referring to a same time reference to generate aggregate output values for each time reference, storing the aggregate output values in a segment identification array corresponding to the predefined portion of the waveform data. In a seventh example of the method, optionally including one or more or each of the first through sixth examples, aggregating the individual output values further comprises: calculating an average of the individual output values, in response to the average being greater than a threshold value, assigning a one as an aggregate output value, and in response to the average being less than the threshold value, assigning a zero as the aggregate output value. In a eighth example of the method, optionally including one or more or each of the first through seventh examples, an amount of respective chunk segment identification arrays over which the individual output values are aggregated varies based on a time-alignment of the respective chunk segment identification arrays. In a ninth example of the method, optionally including one or more or each of the first through eighth examples, displaying the location of the segment including the specified event on the display device further comprises: detecting a first starting point and a first ending point of a consecutive number of ones bounded by zeros in the segment identification array corresponding to the predefined portion of the waveform data, displaying a plot of the predefined portion of the waveform data, and indicating a second starting point and second ending point of the segment on the plot, the second starting point based on the first starting point, and the second ending point based on the first ending point.

The disclosure also provides support for a method for a waveform processing system, the method comprising: after a first plurality of time instances of a first time interval, inputting waveform data collected at each time instance of the first plurality of time instances into an artificial intelligence (al) model to generate a first output array of values, after a second plurality of time instances of a second time interval, the second time interval having a length equal to the first time interval, inputting waveform data collected at each time instance of the second plurality of time instances into the al model to generate a second output array of values, time-aligning the first output array of values and the second output array of values based on a difference in time between a first initial time instance of the first plurality of time instances and a second initial time instance of the second plurality of time instances, aggregating the time-aligned first output array of values and second output array of values to generate a segment identification array of aggregated values, the segment identification array indicating a location of a segment in the waveform data including a predefined event, and displaying the location of the predefined event within the waveform data on a display device of the waveform processing system. In a first example of the method, the method further comprises: after a third plurality of time instances of a third time interval, the third time interval having a length equal to the first and second time intervals, inputting waveform data collected at each time instance of the third plurality of time instances into the AI model to generate a third output array of values, time-aligning the first output array of values, the second output array of values, and the third output array of values based on a difference in time between a third initial time instance of the third plurality of time instances and the second initial time instance of the second plurality of time instances, and aggregating the time-aligned first output array of values, second output array of values, and third output array of values to generate the segment identification array of aggregated values. In a second example of the method, optionally including the first example, the method further comprises: continuing to aggregate additional time-aligned output arrays of values to generate the segment identification array of aggregated values, the additional time-aligned output arrays of values generated by inputting waveform data collected at each time instance of additional pluralities of time instances of additional time intervals into the AI model until a length of the segment identification array is equal to a length of a waveform data array including a predefined amount of waveform data. In a third example of the method, optionally including one or both of the first and second examples, the values of the output arrays of values are between zero and one, and aggregating the time-aligned output arrays of values to generate the segment identification array of aggregated values further comprises aggregating individual output values of the time-aligned output arrays of values referring to a same time instance to generate aggregated output values for each time instance. In a fourth example of the method, optionally including one or more or each of the first through third examples, aggregating the individual output values further comprises: calculating an average output value of the individual output values, in response to the average output value being greater than or equal to a threshold value, assigning a one to a position in the segment identification array corresponding to the average output value, and in response to the average being less than the threshold value, assigning a zero to the position in the segment identification array corresponding to the average output value. In a fifth example of the method, optionally including one or more or each of the first through fourth examples,: a first number of the individual output values that are averaged at a first time instance of the first plurality of time instances is one, a second number of the individual output values that are averaged at a last time instance of a last plurality of time instances is one, and a maximum number of individual output values are averaged at a time instance in a middle of the predefined amount of waveform data. In a sixth example of the method, optionally including one or more or each of the first through fifth examples, displaying the location of the predefined event within the waveform data on the display device further comprises: detecting a consecutive number of ones bounded by zeros in the segment identification array, determining a first position of the segment identification array corresponding to a starting point of the consecutive number of ones, determining a second position of the segment identification array corresponding to an ending point of the consecutive number of ones, displaying the waveform data of the waveform data array on the display device, indicating a start of the location of the predefined event in the displayed waveform data based on the first position, and indicating an end of the location of the predefined event in the displayed waveform data based on the second position.

The disclosure also provides support for a waveform processing system, comprising: a display device, a processor, and a non-transitory memory including instructions that when executed, cause the processor to display a location of a segment including a specified event in a stream of waveform data on the display device based on a segment identification array, the segment identification array generated by: dividing the waveform data into a plurality of overlapping chunks of a fixed length, where each chunk is offset from a preceding chunk by a predefined inference interval, at each inference interval, inputting a plurality of data values of each overlapping chunk of the waveform data into an artificial intelligence (aI) model, the al model trained to output a chunk segment identification array including a respective plurality of output values, each output value of the respective plurality of output values indicating a presence or absence of a specified event in the overlapping chunk of the waveform data, aggregating a plurality of chunk segment identification arrays outputted by the al model for a corresponding plurality of overlapping chunks to create the segment identification array. In a first example of the system, aggregating the plurality of chunk segment identification arrays outputted by the AI model further comprises: time-aligning the chunk segment identification arrays, averaging individual output values of the time-aligned chunk segment identification arrays associated with a same time reference to generate average output values for the time reference, assigning a first value to the segment identification array for each average value greater than or equal to a threshold value, the first value indicating a presence of the segment, and assigning a second value to the segment identification array for each average value less than threshold value, the second value indicating an absence of the segment. In a second example of the system, optionally including the first example,: a number of the individual output values aggregated to generate the segment identification array increases with each time reference from one individual output value at a beginning of the segment identification array, to a maximum number of individual output values, and the number of the individual output values aggregated to generate the segment identification array decreases with each time reference from the maximum number of individual output values to one individual output value at an end of the segment identification array.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method for a waveform processing system, the method comprising:
displaying, on a display device of the waveform processing system (718), a location in a stream of waveform data of a segment including a specified event, wherein the location of the segment is identified based on aggregating outputs of an AI model (710) performing sliding window inferences on the stream of waveform data.

2. The method of claim 1, wherein the specified event is an aberration or variation in a plot of waveform data from an expected plot.

3. The method of claim 1, wherein performing the sliding window inferences on the stream of waveform data further comprises:
dividing the waveform data into a plurality of overlapping chunks (706), each overlapping chunk comprising an array of consecutive data values extracted from the stream of waveform data over a specified duration, the array having a predefined size;
at a predefined inference interval, inputting the array of consecutive data values of a chunk of the overlapping chunks of waveform data into the AI model (708), and receiving an output of the AI model, the output comprising a respective chunk segment identification array of output values of the predefined size, the output values indicating the location of the segment in the chunk of the waveform data.

4. The method of claim 3, wherein a start of each overlapping chunk of the plurality of overlapping chunks is offset from a start of a preceding overlapping chunk by the predefined inference interval.

5. The method of claim 3, wherein the respective chunk segment identification array includes a plurality of ones and zeros, where a one in a position of the chunk segment identification array indicates that a data value in a corresponding position of the inputted array of consecutive data values is included in the segment, and a zero in a position of the chunk segment identification array indicates that a data value in a corresponding position of the array of consecutive data values is not included in the segment.

6. The method of claim 3, wherein the specified duration of each overlapping chunk is longer than the predefined inference interval.

7. The method of claim 3, wherein aggregating the outputs of the AI model further comprises:
for a predefined portion of the waveform data:
time-aligning a plurality of respective chunk segment identification arrays outputted by the AI model corresponding to the predefined portion (712);
aggregating individual output values of the time-aligned chunk segment identification arrays referring to a same time reference to generate aggregate output values for each time reference (714);
storing the aggregate output values in a segment identification array corresponding to the predefined portion of the waveform data.

8. The method of claim 7, wherein aggregating the individual output values further comprises:
calculating an average of the individual output values;
in response to the average being greater than a threshold value, assigning a one as an aggregate output value; and
in response to the average being less than the threshold value, assigning a zero as the aggregate output value.

9. The method of claim 7, wherein an amount of respective chunk segment identification arrays over which the individual output values are aggregated varies based on a time-alignment of the respective chunk segment identification arrays.

10. The method of claim 7, wherein displaying the location of the segment including the specified event on the display device (718) further comprises:
detecting a first starting point and a first ending point of a consecutive number of ones bounded by zeros in the segment identification array corresponding to the predefined portion of the waveform data;
displaying a plot of the predefined portion of the waveform data; and
indicating a second starting point and second ending point of the segment on the plot, the second starting point based on the first starting point, and the second ending point based on the first ending point.

11. A waveform processing system (102), comprising:
a display device (134);
a processor (104); and
a non-transitory memory (106) including instructions that when executed, cause the processor (104) to display a location of a segment including a specified event in a stream of waveform data (252) on the display device(134) based on a segment identification array (262), the segment identification array (262) generated by:
dividing the waveform data into a plurality of overlapping chunks (401) of a fixed length, where each chunk (401) is offset from a preceding chunk by a predefined inference interval;
at each inference interval, inputting a plurality of data values of each overlapping chunk of the waveform data into an artificial intelligence (AI) model (230), the AI model (230) trained to output a chunk segment identification array (258) including a respective plurality of output values, each output value of the respective plurality of output values indicating a presence or absence of a specified event in the overlapping chunk (401) of the waveform data; and
aggregating a plurality of chunk segment identification arrays (462) outputted by the AI model for a corresponding plurality of overlapping chunks (401) to create the segment identification array (262).

12. The waveform processing system of claim 11, wherein aggregating the plurality of chunk segment identification arrays (462) outputted by the AI model (230) further comprises:
time-aligning the chunk segment identification arrays (462);
averaging individual output values of the time-aligned chunk segment identification arrays (462) associated with a same time reference to generate average output values for the time reference;
assigning a first value to the segment identification array (262) for each average value greater than or equal to a threshold value, the first value indicating a presence of the segment; and
assigning a second value to the segment identification array (262) for each average value less than threshold value, the second value indicating an absence of the segment.

13. The waveform processing system of claim 11, wherein:
a number of the individual output values aggregated to generate the segment identification array (262) increases with each time reference from one individual output value at a beginning of the segment identification array (262), to a maximum number of individual output values; and
the number of the individual output values aggregated to generate the segment identification array (262) decreases with each time reference from the maximum number of individual output values to one individual output value at an end of the segment identification array (262).
